# EUROPEAN PATENT APPLICATION

(11) **EP 4 778 888 A1**
(43) Date of publication of application: **22.07.2026**
(21) Application number: 23952130.5
(22) Date of filing: 28.12.2023
(51) Int. Cl.: C01B 39/02, C01B 39/04

(54) **MWW-TYPE MOLECULAR SIEVE, AND PREPARATION METHOD THEREFOR AND USE THEREOF**

(30) Priority: 12.09.2023 CN 202311174096
(71) Applicant: CHINA PETROLEUM & CHEMICAL CORPORATION, Chaoyang District Beijing 100728 (CN); Sinopec (Shanghai) Research Institute of Petrochemical Technology Co., Ltd., Shanghai 201208 (CN)
(72) Inventor: WANG, Yiyan, Shanghai 201208 (CN); WANG, Darui, Shanghai 201208 (CN); YANG, Weimin, Shanghai 201208 (CN); SUN, Hongmin, Shanghai 201208 (CN); SHEN, Zhenhao, Shanghai 201208 (CN); HE, Junlin, Shanghai 201208 (CN)
(74) Representative: Hoffmann Eitle
(86) International application number: PCT/CN2023/142567
(87) International publication number: WO 2025/055212

(57) **Abstract**

Disclosed are an MWW-type molecular sieve, and a preparation method therefor and a use thereof. The weight ratio of framework aluminum (Al_{[F]}) to extra-framework aluminum (Al_{[EF]}) of the molecular sieve is 5.0-16.0:1. The present invention provides a novel MWW-type molecular sieve. When the catalyst prepared by using the molecular sieve of the present invention is used in a reaction for preparing cyclohexylbenzene by means of transalkylation of polycyclohexylbenzene and benzene, the conversion rate of polycyclohexylbenzene and the selectivity of a cyclohexylbenzene product can be improved.

## Description

### Technical Field

The invention belongs to the technical field of catalytic materials, and in particular relates to an MWW-type molecular sieve, a preparation method therefor and an application thereof.

### Background Art

MCM-22 is a typical MWW-type layered zeolite, which has two sets of independent channel systems that are not connected with each other. One set is an in-layer, two-dimensional sinusoidal channel system composed of 10-membered rings, and the other set is composed of interlayer 12-membered ring supercages, which are connected to each other by very short 10-membered ring channels and is connected to the outside in the form of 10-membered ring openings or a half of 12-membered ring supercage. At present, MCM-22 molecular sieves have been widely used in reactions such as isomerization (CN115770611A), catalytic cracking (US4,983,276) and alkylation (US4,992,615). Studies have revealed that their catalytic performance is closely related to the distribution of acidic sites. Since big molecules such as aromatics are more likely to react in the "half of 12-membered ring supercage" on the outer surface, the distribution of acidic sites on the accessible outer surface has a significant effect on the catalytic performance of the molecular sieves, and the placement of aluminum in the molecular sieve framework is of great significance to the optimization of the catalytic performance of the molecular sieves.

A preparation method of MCM-22 molecular sieve is first reported by Mobil (US4,954,325), using hexamethyleneimine as a template and silicon dioxide, sodium aluminate, sodium hydroxide and deionized water as raw materials. In order to further improve the catalytic performance, a variety of treatment methods have been developed, such as interlayer swelling or pore expansion by dissolving silicon or dealumination, to increase the exposure of active sites.

The method disclosed in WO1997017290A is to mix MCM-22 molecular sieve slurry with a solution of cetyltrimethylammonium hydroxide and tetrapropylammonium hydroxide of specific concentrations, stir to obtain a swollen MCM-22 molecular sieve, and then ultrasonically treat to obtain an ITQ-2 molecular sieve having a single-layer structure of MCM-22.

CN103803577A discloses a small-grain ultra-thin MCM-22 molecular sieve and a preparation method thereof. The method introduces heavy water into a hydrothermal synthesis system to control the growth of the molecular sieve in length and thickness directions, thereby obtaining the small-grain ultra-thin MCM-22 molecular sieve.

The above molecular sieves each have their own characteristics, and the development and research of new MWW-type molecular sieves with specific structures and properties are still ongoing.

### Disclosure of the invention

The purpose of the present invention is to provide a new MWW-type molecular sieve, a preparation method therefor and an application thereof. When a catalyst prepared from the MWW-type molecular sieve of the present invention is used in the transalkylation of a polycyclohexylbenzene and benzene, the conversion rate of the polycyclohexylbenzene and the selectivity of the cyclohexylbenzene can be improved.

A first aspect of the present invention provides an MWW-type molecular sieve, wherein a weight ratio of framework aluminum (Al_{[F]}) to extra-framework aluminum (Al_{[EF]}) ranges from 5.0:1 to 16.0:1.

In some embodiments, the molecular sieve has a weight ratio of framework aluminum (Al_{[F]}) to extra-framework aluminum (Al_{[EF]}) of from 5.0:1 to 16.0:1, and preferably from 6.5:1 to 10.5:1, for example, 5.0, 5.5, 6.0, 6.5, 7.0, 7.5, 8.0, 8.5, 9.0, 9.5, 10.0, 10.5, 11.0, 12.0, 13.0, 14.0, 15.0, or 16.0.

In some embodiments, the molecular sieve has a weight ratio of aluminum in the semi-supercages on its outer surface (Al₍₆₁₎) to framework aluminum (Al_{[F]}) of from 0.18:1 to 0.60:1, and preferably from 0.22:1 to 0.50:1, for example, 0.18, 0.20, 0.22, 0.23, 0.24, 0.25, 0.28, 0.30, 0.32, 0.35, 0.38, 0.40, 0.42, 0.45, 0.48, 0.50, 0.55, or 0.60.

In some embodiments, the molecular sieve has a SiO₂/Al₂O₃ molar ratio of from 10 to 60, and preferably from 15 to 50.

In some embodiments, the molecular sieve has a pore volume of micropores (having a pore diameter less than 2 nm) of from 0.15 to 0.25 cm³/g, and preferably from 0.16 to 0.20 cm³/g, a pore volume of mesopores (having a pore diameter greater than or equal to 2 nm and less than 50 nm) of from 0.35 to 0.55 cm³/g, and preferably from 0.40 to 0.50 cm³/g, and a specific surface area of from 400 to 550 m²/g, and preferably from 410 to 500 m²/g.

In some embodiments, the molecular sieve is an MWW-type silica-alumina molecular sieve having a 12-membered ring channel structure, and preferably an MCM-22 molecular sieve.

A second aspect of the present invention provides a method for preparing the above molecular sieve, comprising:
(a) contacting a silicon source, an aluminum source, an alkali source, water, a template agent, and a stabilizer to obtain a mixture A;
(b) crystallizing the mixture A to obtain the molecular sieve.

In some embodiments, the silicon source in step (a) is preferably one or more of tetraethoxysilane, alkaline silica sol, white carbon black, sodium silicate, and silicic acid.

In some embodiments, the aluminum source in step (a) is preferably one or more of sodium aluminate, pseudo-boehmite, aluminum isopropoxide, aluminum sulfate, and aluminum chloride.

In some embodiments, the alkali source in step (a) is preferably one or more of sodium hydroxide and potassium hydroxide.

In some embodiments, the template agent in step (a) is preferably one or more of hexamethyleneimine, piperidine, ethylenediamine, cyclohexylamine, and dimethylcyclohexylamine.

In some embodiments, the stabilizer in step (a) is at least one amide compound of the following formula: 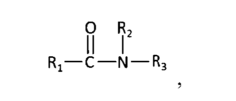 wherein R₁, R₂ and R₃ are each independently selected from the group consisting of hydrogen, C1-C15 alkyl, C5-C15 cycloalkyl, and C4-C15 nitrogen-containing organic groups, and preferably at least one of R₁, R₂ and R₃ is a C5-C10 cycloalkyl or a C4-C9 nitrogen-containing heterocyclic organic group, for example, R₁, R₂ and R₃ are each independently selected from the group consisting of hydrogen, ethyl, cyclohexyl, piperidinyl, pyridinyl, pyrimidinyl, methyl piperidinyl, methyl pyridinyl and methyl pyrimidinyl. Examples of the stabilizer include, but are not limited to, 4-piperidinecarboxamide, N-cyclohexylformamide, 2-pyridinecarboxamide, 6-methylnicotinamide, and N-ethylformamide.

In some preferred embodiments, in step (a), the molar ratio of the silicon source in terms of SiO₂ to the aluminum source in terms of Al₂O₃ to the alkali source to the water to the template agent to the stabilizer is 1 : (0.01~0.10) : (0.02~0.50) : (8~25) : (0.05~0.5) : (0.01~0.30), and preferably 1 : (0.01~0.07) : (0.05~0.30) : (10~20) : (0.1~0.5) : (0.02~0.10).

In some preferred embodiments, conditions for the crystallizing in step (b) include dynamically crystallizing under closed conditions, a crystallization temperature of 90 to 190 °C, and a crystallization time of 20 to 120 hours. The dynamically crystallizing is carried out under stirring conditions, preferably at a stirring speed of 10 to 50 rpm.

In some preferred embodiments, the crystallizing in step (b) may be followed by conventional posttreatments known in the art, including separation, washing, drying and calcination. The separation may be carried out by centrifugal separation, and the washing is washing solids obtained by the above solid-liquid separation with deionized water to a pH value of about 7.0 to 8.0. Conditions for the drying may include a temperature of 100 to 180 °C and a period of time of 5 to 20 hours. Calcination temperature may be 450 to 600 °C, calcination time may be 2 to 6 hours, and calcination atmosphere may be an oxygen-containing gas, such as air.

In some preferred embodiments, the method for preparing the molecular sieve can achieve a utilization rate of the raw aluminum source of 91.0 % to 99.0 %, and preferably 93.0 % to 98.0 %.

A third aspect of the present invention provides a transalkylation catalyst comprising the above molecular sieve.

In some embodiments, the molecular sieve in the catalyst is an H-type molecular sieve. A molecular sieve as-synthesized can be converted into the H-type molecular sieve by a conventional mothed in the art. For example, the molecular sieve as-synthesized can be converted into the H-type molecular sieve separately, and the H-type molecular sieve is then made into a catalyst. It is also possible to first make the molecular sieve as-synthesized into a catalyst and then convert the molecular sieve in the catalyst into the H-type molecular sieve.

In some embodiments, the H-type molecular sieve in the catalyst can be obtained by conventional ammonium exchange and then calcination. The ammonium exchange can be carried out by treating with an ammonium salt solution with a mass concentration of 2% to 10% at 20 to 70 °C for 1 to 5 hours, and the ammonium exchange can be continuously conducted for 2 to 5 times. The ammonium salt is at least one of ammonium sulfate, ammonium acetate and ammonium nitrate. In the ammonium exchange, the amount of the ammonium salt solution used is such that a liquid-to-solid volume ratio ranges from 10 : 1 to 3 : 1. The calcination after the ammonium exchange can be carried out in a conventional manner, for example, calcining at 450 to 600 °C for 2 to 6 hours. This calcination can be accomplished with the calcination comprised in the catalyst preparation in one step.

The catalyst can be in any desired physical form, such as powder, granules or molded products such as tablets, strips, cut three-lobed extrudate with a 3 to 10 mm length. These physical forms can be obtained in any conventional manner known in the art, and there is no particular limitation here. When the catalyst is molded as required, a specific method can be as follows: the above-described molecular sieve is mixed with a binder and molded, followed by drying and calcining, to obtain the catalyst. The binder can be at least one of alumina, silica and titania. The amount of the binder added can account for 10% to 40% of the mass of the molecular sieve.

In some embodiments, during the molding process, molding aids such as cellulose, water, etc. may be added.

In the catalyst preparation method, the drying and the calcining can be carried out in a conventional manner. Preferably, conditions for the drying include a drying temperature of 90 to 160 °C and a drying time of 6 to 15 hours. Conditions for the calcining include an oxygen-containing atmosphere (such as air atmosphere), a calcination temperature of 300 to 550 °C, and a calcination time of 2 to 5 hours.

A fourth aspect of the present invention provides the use of the above-described molecular sieve or the above-described catalyst in the transalkylation of polycyclohexylbenzene and benzene.

Therefore, in some embodiments, the present invention provides a method for preparing cyclohexylbenzene, comprising contacting a polycyclohexylbenzene and benzene with the above catalyst to perform a transalkylation reaction to obtain a reaction effluent comprising cyclohexylbenzene, and separating cyclohexylbenzene from the reaction effluent.

In some embodiments, conditions of the transalkylation reaction include a reaction temperature of 100 to 200 °C, and preferably 135 to 175 °C; a pressure of 0.5 to 2.0 MPa, and preferably 0.7 to 1.5 MPa; a molar ratio of benzene to polycyclohexylbenzene of 1 to 20, and preferably 2 to 10; and a mass liquid hourly space velocity of the total reaction feed of 0.5 to 20 h⁻¹, and preferably 1 to 15 h⁻¹.

In some embodiments, the polycyclohexylbenzene may be at least one of dicyclohexylbenzene and tricyclohexylbenzene.

A fifth aspect of the present invention provides the use of the above-described molecular sieve or the above-described catalyst in the preparation of cyclohexylbenzene via hydroalkylation of benzene.

A hydroalkylation catalyst used in the hydroalkylation reaction of benzene to cyclohexylbenzene comprises the above-described molecular sieve or the above-described catalyst and a hydrogenation metal.

Any known hydrogenation metal can be used in the hydroalkylation catalyst. In some embodiments, the hydrogenation metal component is at least one selected from Ru, Pd, Pt, Ni, Zn, Sn and Co, and preferably from Pd and Ru. The hydroalkylation catalyst may have a content of the hydrogenation metal component of from 0.02 to 5.0 wt%, preferably from 0.02 to 2.0 wt%, more preferably from 0.05 to 1.0 wt%, and even more preferably from 0.08 to 0.25 wt%, based on the total weight of the hydroalkylation catalyst.

Methods for converting the above-described molecular sieves into hydroalkylation catalysts are well known in the art.

Therefore, in some embodiments, the present invention provides a method for preparing cyclohexylbenzene via hydroalkylation of benzene, comprising contacting hydrogen and benzene with the hydroalkylation catalyst under hydroalkylation conditions to produce a reaction effluent comprising cyclohexylbenzene, and separating cyclohexylbenzene from the reaction effluent.

In some embodiments, conditions of the hydroalkylation of benzene include a reaction temperature of 100 to 200 °C, and preferably 130 to 170 °C; a pressure of 0.5 to 2.0 MPa, and preferably 0.7 to 1.5 MPa; a molar ratio of benzene to hydrogen of 1 to 20, and preferably 2 to 10; and a mass liquid hourly space velocity of benzene of 0.5 to 10 h⁻¹, and preferably 1 to 5 h⁻¹.

Compared to the prior art, the present invention has the following advantages:
1. The present invention provides a new MWW-type molecular sieve having a specific content and distribution of the framework aluminum.
2. The inventors have found through research that using a molecular sieve with a specific content and distribution of the framework aluminum as a catalyst in the polycyclohexylbenzene transalkylation reaction can effectively improve the conversion rate of polycyclohexylbenzene and the selectivity of the cyclohexylbenzene product.
3. The present invention can effectively limit and regulate the distribution of framework aluminum in the molecular sieve by introducing a stabilizer during the preparation of the molecular sieve, thereby achieving efficient utilization of active centers in the molecular sieve and improving the performance of the catalyst, especially for the transalkylation of polycyclohexylbenzene and the hydroalkylation of benzene to produce cyclohexylbenzene, in which good cyclohexylbenzene selectivity can be achieved.

### Brief description of the drawings

FIG. 1 shows XRD patterns of the molecular sieves obtained in inventive Example 1 and Comparative Example 1.
FIG. 2 shows an Al-NMR spectrum of the molecular sieve M1 obtained in inventive Example 1.
FIG. 3 shows an Al-NMR spectrum of the molecular sieve D1 obtained in Comparative Example 1.

### Examples

The present invention will be described in detail in conjunction with examples below, in order to more clearly understand the technical features, purposes and beneficial effects of the present invention. However, it should be understood that the protection scope of the present invention is not limited to these examples.

In the present invention, the distribution of aluminum species in the molecular sieve was obtained by analyzing the measurement results of ²⁷Al MAS NMR. The measurement was conducted on a JEOL 500 MHz (11.7T) spectrometer by using a 3.2 mm HX MAS The NMR probe rotating at 18 kHz. The spectra were acquired at a resonance frequency of 130.3 MHz, and the chemical shifts were referenced to 1 mol/L Al(NO₃)₃ solution (δ = 0 ppm). In the spectra, characteristic peaks at δ = 0 ppm and 54 ppm are attributable to octahedrally coordinated extra-framework aluminum (Al_{[EF]}) and tetrahedronally coordinated framework aluminum (Al_{[F]}), respectively. The characteristic peak at δ = 54 ppm was subjected to peak-resolving and fitting calculation to determine the distribution of aluminum at different tetrahedral sites, among which the Al location at δ = 50 ppm (Al₍₅₀₎) corresponds to the Al_{[F]} distribution of the 12MR (i.e., 12-membered ring) supercage of the molecular sieve, the Al location at δ = 56 ppm (Al₍₅₆₎) corresponds to the Al_{[F]} distribution in the sinusoidal channels, and the Al location at δ = 61 ppm (Al₍₆₁₎) corresponds to the Al_{[F]} distribution of the 12MR semi-supercage on the outer surface.

In the present invention, a Micromeritics ASAP-2000 physical adsorption instrument was used to measure the N₂ adsorption-desorption isotherm at 77K. Prior to the measurement, samples were degassed under vacuum at 573K for 4h. Total pore volume and specific surface area are calculated by using the BET equation, and micropore volume is determined by using the t-plot curve method. The difference between the total pore volume and the micropore volume is the mesopore volume.

In the present invention, the SiO₂/Al₂O₃ molar ratios of the molecular sieves were obtained by ICP measurement conducted on a Varian 725-ES ICP-AES analyzer.

### Example 1

This example illustrates the synthesis of a transalkylation catalyst.
(1) 11.3 g of sodium aluminate (having a content of Al₂O₃ of 45.0 wt%) and 6.0 g of sodium hydroxide were added into 234.0 g of water and continuously stirred until solids were dissolved. Then, 24.8 g of hexamethyleneimine and 6.4 g of 4-piperidinecarboxamide were added into the solution. After stirring for 30 min, 208.3 g of tetraethoxysilane was added thereinto. Thus, the molar ratio of the reactants, tetraethoxysilane in terms of SiO₂ : sodium aluminate in terms of AL₂O₃ : NaOH : water : hexamethyleneimine : 4-piperidinecarboxamide, was 1 : 0.05 : 0.15 : 13 : 0.25 : 0.05. The stirring continued for 30 min to uniformly mix the mixture, and the resulting mixture was then placed into a stainless-steel reactor with a polytetrafluoroethylene liner and allowed to crystallize at 160 °C and 30 rpm for 72 hours. The reaction mixture was centrifuged, and the obtained solids were washed with deionized water to pH 7, dried in an oven at 150 °C for 8 h, and calcined under air atmosphere at 500 °C for 5 h, to obtain a molecular sieve M1.
(2) 10 g of the molecular sieve M1 was mixed uniformly with 2.0 g of silica, 0.1 g of cellulose and 5.0 g of deionized water, and the resulting mixture was kneaded and molded into strips and then cut into pellets. The pellets were dried at a constant temperature of 120 °C for 12h. Then, the dried pellets were treated with an ammonium acetate solution with 5% mass concentration at a liquid-to-solid volume ratio of 10 : 1 at 30 °C for 2h. This exchange treatment was conducted three times in total. The exchange treated solids were dried at a constant temperature of 150 °C for 12h and then calcined under air atmosphere at 500 °C for 3h, to obtain a target catalyst C1.

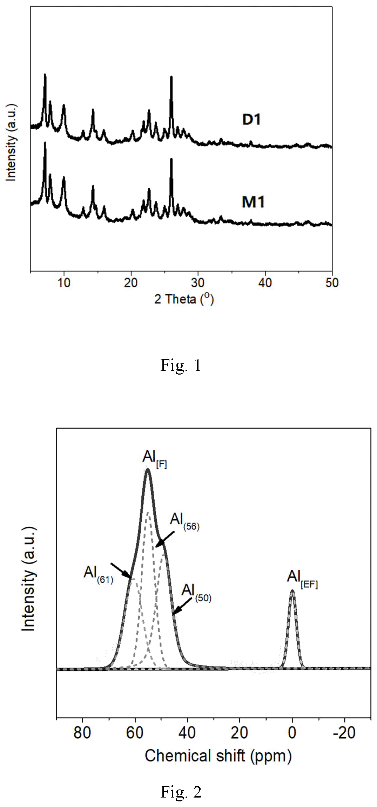

An XRD pattern of the molecular sieve M1 is shown in FIG. 1, from which it can be seen that it is an MCM-22 molecular sieve.

An Al-NMR spectrum of the molecular sieve M1 is shown in FIG. 2, with the distribution of aluminum species being shown in Table 1 below.

The utilization rate of the raw material aluminum source in the preparation of the molecular sieve M1 is shown in Table 1 below.

Measurements revealed that the molecular sieve M1 had a micropore volume of 0.19 cm³/g, a mesopore volume of 0.50 cm³/g, and a specific surface area of 483 m²/g.

The catalyst C1 was evaluated in a transalkylation reaction of polycyclohexylbenzene and benzene, wherein the reaction temperature was 150 °C, the reaction pressure was 1.0 MPa, the molar ratio of benzene to dicyclohexylbenzene was 3, and the mass liquid hourly space velocity of the total feed was 10 h⁻¹. The results after 24 hours of reaction are shown in Table 2 below.

### Example 2

(1) 9.2 g of sodium aluminate (having a content of Al₂O₃ of 45.0 wt%) and 2.0 g of sodium hydroxide were added into 360.0 g of water and continuously stirred until solids were dissolved. Then, 70.1 g of hexamethyleneimine and 9.8 g of 2-pyridinecarboxamide were added into the solution. After stirring for 30 min, 208.3 g of tetraethoxysilane was added thereinto. Thus, the molar ratio of the reactants, tetraethoxysilane in terms of SiO₂ : sodium aluminate in terms of AL₂O₃ : NaOH : water : hexamethyleneimine : 2-pyridinecarboxamide, was 1 : 0.04 : 0.05 : 20 : 0.5 : 0.08. The stirring continued for 30 min to uniformly mix the mixture, and the resulting mixture was then placed into a stainless-steel reactor with a polytetrafluoroethylene liner and allowed to crystallize at 170 °C and 20 rpm for 60 hours. The reaction mixture was centrifuged, and the obtained solids were washed with deionized water to pH 7, dried in an oven at 130 °C for 10 h, and calcined under air atmosphere at 550 °C for 4 h, to obtain a molecular sieve M2.
(2) 10 g of the molecular sieve M2 was mixed uniformly with 2.5 g of silica, 0.1 g of cellulose and 5.0 g of deionized water, and the resulting mixture was kneaded and molded into trilobal strips and then cut into pellets. The pellets were dried at a constant temperature of 110 °C for 10h. Then, the dried pellets were treated with an ammonium sulfate solution with 7% mass concentration at a liquid-to-solid volume ratio of 8 : 1 at 20 °C for 1h. This exchange treatment was conducted four times in total. The exchange treated solids were dried at a constant temperature of 150 °C for 12h and then calcined under air atmosphere at 500 °C for 4h, to obtain a target catalyst C2.

An XRD pattern of the molecular sieve M2 showed that it was an MCM-22 molecular sieve. Al-NMR measurement was conducted on the molecular sieve M2, and the distribution of aluminum species obtained through peak-resolving and fitting calculation is shown in Table 1 below.

The utilization rate of the raw material aluminum source in the preparation of the molecular sieve M2 is shown in Table 1 below.

Measurements revealed that the molecular sieve M2 had a micropore volume of 0.17 cm³/g, a mesopore volume of 0.45 cm³/g, and a specific surface area of 449 m²/g.

The catalyst C2 was evaluated in a transalkylation reaction of polycyclohexylbenzene and benzene, wherein the reaction temperature was 150 °C, the reaction pressure was 1.0 MPa, the molar ratio of benzene to dicyclohexylbenzene was 3, and the mass liquid hourly space velocity of the total feed was 10 h⁻¹. The results after 24 hours of reaction are shown in Table 2 below.

### Example 3

(1) 6.8 g of sodium aluminate (having a content of Al₂O₃ of 45.0 wt%) and 8.0 g of sodium hydroxide were added into 270.0 g of water and continuously stirred until solids were dissolved. Then, 17.0 g of piperidine and 4.1 g of 6-methylnicotinamide were added into the solution. After stirring for 30 min, 208.3 g of tetraethoxysilane was added thereinto. Thus, the molar ratio of the reactants, tetraethoxysilane in terms of SiO₂ : sodium aluminate in terms of Al₂O₃ : NaOH : water : piperidine : stabilizer, was 1 : 0.03 : 0.2 : 15 : 0.2 : 0.03. The stirring continued for 30 min to uniformly mix the mixture, and the resulting mixture was then placed into a stainless-steel reactor with a polytetrafluoroethylene liner and allowed to crystallize at 150 °C and 25 rpm for 96 hours. The reaction mixture was centrifuged, and the obtained solids were washed with deionized water to pH 7, dried in an oven at 140 °C for 9 h, and calcined under air atmosphere at 550 °C for 6 h, to obtain a molecular sieve M3.
(2) 10 g of the molecular sieve M3 was mixed uniformly with 1.5 g of silica, 0.1 g of cellulose and 5.0 g of deionized water, and the resulting mixture was kneaded and molded into strips and then cut into pellets. The pellets were dried at a constant temperature of 100 °C for 10h. Then, the dried pellets were treated with an ammonium acetate solution with 8% mass concentration at a liquid-to-solid volume ratio of 6 : 1 at 40 °C for 1h. This exchange treatment was conducted four times in total. The exchange treated solids were dried at a constant temperature of 150 °C for 12h and then calcined under air atmosphere at 500 °C for 2h, to obtain a target catalyst C3.

An XRD pattern of the molecular sieve M3 showed that it was an MCM-22 molecular sieve.

Al-NMR measurement was conducted on the molecular sieve M3, and the distribution of aluminum species obtained through peak-resolving and fitting calculation is shown in Table 1 below.

The utilization rate of the raw material aluminum source in the preparation of the molecular sieve M3 is shown in Table 1 below.

Measurements revealed that the molecular sieve M3 had a micropore volume of 0.17 cm³/g, a mesopore volume of 0.41 cm³/g, and a specific surface area of 435 m²/g.

The catalyst C3 was evaluated in a transalkylation reaction of polycyclohexylbenzene and benzene, wherein the reaction temperature was 150 °C, the reaction pressure was 1.0 MPa, the molar ratio of benzene to dicyclohexylbenzene was 3, and the mass liquid hourly space velocity of the total feed was 10 h⁻¹. The results after 24 hours of reaction are shown in Table 2 below.

### Example 4

(1) 5.8 g of pseudo-boehmite and 8.4 g of potassium hydroxide were added into 216.0 g of water and continuously stirred until solids were dissolved. Then, 24.8 g of hexamethyleneimine and 4.4 g of N-ethylformamide were added into the solution. After stirring for 30 min, 150.0 g of silica sol was added thereinto. Thus, the molar ratio of the reactants, silica sol in terms of SiO₂ : pseudo-boehmite in terms of Al₂O₃ : KOH : water : hexamethyleneimine : stabilizer, was 1 : 0.05 : 0.15 : 12 : 0.25 : 0.06. The stirring continued for 30 min to uniformly mix the mixture, and the resulting mixture was then placed into a stainless-steel reactor with a polytetrafluoroethylene liner and allowed to crystallize at 175 °C and 15 rpm for 40 hours. The reaction mixture was centrifuged, and the obtained solids were washed with deionized water to pH 7, dried in an oven at 120 °C for 12 h, and calcined under air atmosphere at 480 °C for 5 h, to obtain a molecular sieve M4.
(2) 10 g of the molecular sieve M4 was mixed uniformly with 1.0 g of silica, 0.1 g of cellulose and 5.0 g of deionized water, and the resulting mixture was kneaded and molded into strips and then cut into pellets. The pellets were dried at a constant temperature of 120 °C for 8 h. Then, the dried pellets were treated with an ammonium acetate solution with 9% mass concentration at a liquid-to-solid volume ratio of 5 : 1 at 30 °C for 1h. This exchange treatment was conducted four times in total. The exchange treated solids were dried at a constant temperature of 150 °C for 12h and then calcined under air atmosphere at 500 °C for 2h, to obtain a target catalyst C4.

An XRD pattern of the molecular sieve M4 showed that it was an MCM-22 molecular sieve.

Al-NMR measurement was conducted on the molecular sieve M4, and the distribution of aluminum species obtained through peak-resolving and fitting calculation is shown in Table 1 below.

The utilization rate of the raw material aluminum source in the preparation of the molecular sieve M4 is shown in Table 1 below.

Measurements revealed that the molecular sieve M4 had a micropore volume of 0.18 cm³/g, a mesopore volume of 0.43 cm³/g, and a specific surface area of 455 m²/g.

The catalyst C4 was evaluated in a transalkylation reaction of polycyclohexylbenzene and benzene, wherein the reaction temperature was 150 °C, the reaction pressure was 1.0 MPa, the molar ratio of benzene to dicyclohexylbenzene was 3, and the mass liquid hourly space velocity of the total feed was 10 h⁻¹. The results after 24 hours of reaction are shown in Table 2 below.

### Example 5

(1) 10.2 g of aluminum isopropoxide and 6.0 g of NaOH were added into 324.0 g of water and continuously stirred until solids were dissolved. Then, 24.8 g of hexamethyleneimine and 6.4 g of N-cyclohexylformamide were added into the solution. After stirring for 30 min, 65.0 g of white carbon black was added thereinto. Thus, the molar ratio of the reactants, white carbon black in terms of SiO₂ : aluminum isopropoxide in terms of Al₂O₃ : NaOH : water : hexamethyleneimine : stabilizer, was 1 : 0.05 : 0.2 : 18 : 0.25 : 0.05. The stirring continued for 30 min to uniformly mix the mixture, and the resulting mixture was then placed into a stainless-steel reactor with a polytetrafluoroethylene liner and allowed to crystallize at 160 °C and 30 rpm for 72 hours. The reaction mixture was centrifuged, and the obtained solids were washed with deionized water to pH 7, dried in an oven at 150 °C for 8 h, and calcined under air atmosphere at 500 °C for 5 h, to obtain a molecular sieve M5.
(2) 10 g of the molecular sieve M5 was mixed uniformly with 1.5 g of silica, 0.1 g of cellulose and 5.0 g of deionized water, and the resulting mixture was kneaded and molded into strips and then cut into pellets. The pellets were dried at a constant temperature of 120 °C for 12 h. Then, the dried pellets were treated with an ammonium acetate solution with 5% mass concentration at a liquid-to-solid volume ratio of 10 : 1 at 30 °C for 2 h. This exchange treatment was conducted three times in total. The exchange treated solids were dried at a constant temperature of 150 °C for 12 h and then calcined under air atmosphere at 500 °C for 3 h, to obtain a target catalyst C5.

An XRD pattern of the molecular sieve M5 showed that it was an MCM-22 molecular sieve.

Al-NMR measurement was conducted on the molecular sieve M5, and the distribution of aluminum species obtained through peak-resolving and fitting calculation is shown in Table 1 below.

The utilization rate of the raw material aluminum source in the preparation of the molecular sieve M5 is shown in Table 1 below.

Measurements revealed that the molecular sieve M5 had a micropore volume of 0.18 cm³/g, a mesopore volume of 0.46 cm³/g, and a specific surface area of 457 m²/g.

The catalyst C5 was evaluated in a transalkylation reaction of polycyclohexylbenzene and benzene, wherein the reaction temperature was 150 °C, the reaction pressure was 1.0 MPa, the molar ratio of benzene to dicyclohexylbenzene was 3, and the mass liquid hourly space velocity of the total feed was 10 h⁻¹. The results after 24 hours of reaction are shown in Table 2 below.

### Example 6

11.3 g of sodium aluminate (having a content of Al₂O₃ of 45.0 wt%) and 6.0 g of NaOH were added into 234.0 g of water and continuously stirred until solids were dissolved. Then, 24.8 g of hexamethyleneimine and 6.4 g of 4-piperidinecarboxamide were added into the solution. After stirring for 30 min, 208.3 g of tetraethoxysilane was added thereinto. Thus, the molar ratio of the reactants, tetraethoxysilane in terms of SiO₂ : sodium aluminate in terms of Al₂O₃ : NaOH : water : hexamethyleneimine : 4-piperidinecarboxamide, was 1 : 0.05 : 0.15 : 13 : 0.25 : 0.05. The stirring continued for 30 min to uniformly mix the mixture, and the resulting mixture was then placed into a stainless-steel reactor with a polytetrafluoroethylene liner and allowed to crystallize at 160 °C and 30 rpm for 72 hours. The reaction mixture was centrifuged, and the obtained solids were washed with deionized water to pH 7, dried in an oven at 150 °C for 8 h, and calcined under air atmosphere at 500 °C for 5 h, to obtain a molecular sieve M6.

10 g of the molecular sieve M6 was mixed uniformly with 1.0 g of silica, 0.1 g of cellulose and 5.0 g of deionized water, and the resulting mixture was kneaded and molded into strips and then cut into pellets. The pellets were treated with an ammonium acetate solution with 5% mass concentration at a liquid-to-solid volume ratio of 10 : 1 at 30 °C for 2 h. This exchange treatment was conducted three times in total. The exchange treated solids were dried at a constant temperature of 150 °C for 12 h and then calcined under air atmosphere at 500 °C for 3 h, to obtain a target catalyst C6.

An XRD pattern of the molecular sieve M6 showed that it was an MCM-22 molecular sieve.

Al-NMR measurement was conducted on the molecular sieve M6, and the distribution of aluminum species obtained through peak-resolving and fitting calculation is shown in Table 1 below.

The utilization rate of the raw material aluminum source in the preparation of the molecular sieve M6 is shown in Table 1 below.

Measurements revealed that the molecular sieve M6 had a micropore volume of 0.19 cm³/g, a mesopore volume of 0.43 cm³/g, and a specific surface area of 462 m²/g.

The catalyst C6 was evaluated in a transalkylation reaction of polycyclohexylbenzene and benzene, wherein the reaction temperature was 150 °C, the reaction pressure was 1.0 MPa, the molar ratio of benzene to dicyclohexylbenzene was 3, and the mass liquid hourly space velocity of the total feed was 10 h⁻¹. The results after 24 hours of reaction are shown in Table 2 below.

### Example 7

(1) 11.3 g of sodium aluminate (having a content of Al₂O₃ of 45.0 wt%) and 6.0 g of NaOH were added into 234.0 g of water and continuously stirred until solids were dissolved. Then, 24.8 g of hexamethyleneimine and 15.4 g of 4-piperidinecarboxamide were added into the solution. After stirring for 30 min, 208.3 g of tetraethoxysilane was added thereinto. Thus, the molar ratio of the reactants, tetraethoxysilane in terms of SiO₂ : sodium aluminate in terms of AL₂O₃ : NaOH : water : hexamethyleneimine : 4-piperidinecarboxamide, was 1 : 0.05 : 0.15 : 13 : 0.25 : 0.12. The stirring continued for 30 min to uniformly mix the mixture, and the resulting mixture was then placed into a stainless-steel reactor with a polytetrafluoroethylene liner and allowed to crystallize at 160 °C and 30 rpm for 72 hours. The reaction mixture was centrifuged, and the obtained solids were washed with deionized water to pH 7, dried in an oven at 150 °C for 8 h, and calcined under air atmosphere at 500 °C for 5 h, to obtain a molecular sieve M7.
(2) 10 g of the molecular sieve M7 was mixed uniformly with 2.0 g of silica, 0.1 g of cellulose and 5.0 g of deionized water, and the resulting mixture was kneaded and molded into strips and then cut into pellets. The pellets were dried at a constant temperature of 120 °C for 12h. Then, the pellets were treated with an ammonium acetate solution with 5% mass concentration at a liquid-to-solid volume ratio of 10 : 1 at 30 °C for 2 h. This exchange treatment was conducted three times in total. The exchange treated solids were dried at a constant temperature of 150 °C for 12 h and then calcined under air atmosphere at 500 °C for 3 h, to obtain a target catalyst C7.

An XRD pattern of the molecular sieve M7 showed that it was an MCM-22 molecular sieve.

Al-NMR measurement was conducted on the molecular sieve M7, and the distribution of aluminum species obtained through peak-resolving and fitting calculation is shown in Table 1 below.

The utilization rate of the raw material aluminum source in the preparation of the molecular sieve M7 is shown in Table 1 below.

Measurements revealed that the molecular sieve M7 had a micropore volume of 0.16 cm³/g, a mesopore volume of 0.39 cm³/g, and a specific surface area of 410 m²/g.

The catalyst C7 was evaluated in a transalkylation reaction of polycyclohexylbenzene and benzene, wherein the reaction temperature was 150 °C, the reaction pressure was 1.0 MPa, the molar ratio of benzene to dicyclohexylbenzene was 3, and the mass liquid hourly space velocity of the total feed was 10 h⁻¹. The results after 24 hours of reaction are shown in Table 2 below.

### Example 8

The catalyst C1 prepared in Example 1 was loaded with 0.18 % by mass of metal Ru and then used in a hydroalkylation reaction of benzene to produce cyclohexylbenzene, wherein the reaction temperature was 150 °C, the reaction pressure was 0.9 MPa, the mass space velocity of benzene was 1.2 h⁻¹, and the molar ratio of benzene to hydrogen was 2.5. The conversion rate of benzene and the content of key products were determined. After 40 hours of reaction, the conversion rate of benzene was 53.5%, and the selectivity of cyclohexylbenzene was 75.6%.

### Comparative Example 1

Molecular sieve D1 and catalyst C8 were prepared successively by following the procedure described in Example 1, except that the stabilizer, 4-piperidinecarboxamide, was not added.

An XRD pattern of the molecular sieve D1 is shown in Figure 1.

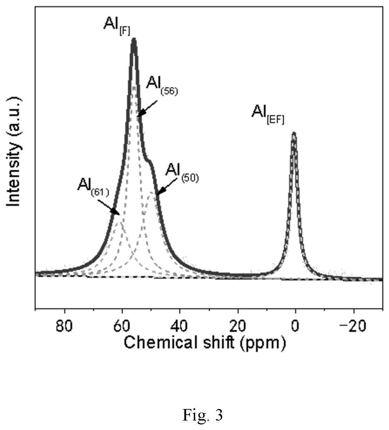

An Al-NMR spectrum of the molecular sieve D1 is shown in Figure 3, and the distribution of aluminum species obtained through peak-resolving and fitting calculation is shown in Table 1 below.

The utilization rate of the raw material aluminum source in the preparation of the molecular sieve D1 is shown in Table 1 below.

Measurements revealed that the molecular sieve D1 had a micropore volume of 0.16 cm³/g, a mesopore volume of 0.42 cm³/g, and a specific surface area of 415 m²/g.

The catalyst C8 was evaluated in a transalkylation reaction of polycyclohexylbenzene and benzene, wherein the reaction temperature was 150 °C, the reaction pressure was 1.0 MPa, the molar ratio of benzene to dicyclohexylbenzene was 3, and the mass liquid hourly space velocity of the total feed was 10 h⁻¹. The results after 24 hours of reaction are shown in Table 2 below.

### Comparative Example 2

Molecular sieve D2 and catalyst C9 were prepared successively by following the procedure described in Example 1, except that the stabilizer, 4-piperidinecarboxamide, was replaced with an equal amount of urea.

An XRD pattern of the molecular sieve D2 showed that it was an MCM-22 molecular sieve.

Al-NMR measurement was conducted on the molecular sieve D2, and the distribution of aluminum species obtained through peak-resolving and fitting calculation is shown in Table 1 below.

The utilization rate of the raw material aluminum source in the preparation of the molecular sieve D2 is shown in Table 1 below.

The catalyst C9 was evaluated in a transalkylation reaction of polycyclohexylbenzene and benzene, wherein the reaction temperature was 150 °C, the reaction pressure was 1.0 MPa, the molar ratio of benzene to dicyclohexylbenzene was 3, and the mass liquid hourly space velocity of the total feed was 10 h⁻¹. The results after 24 hours of reaction are shown in Table 2 below.

**Table 1. Distribution of aluminum species in the molecular sieves obtained in the examples, silica-alumina ratio and utilization rate of raw aluminum source in the molecular sieve preparation**

| Molecular Sieve No. | Framework Aluminum/Extra-framework Aluminum | Semi-supercage Aluminum / Framework Aluminum | SiO₂/Al₂O₃ Molar Ratio | Utilization Rate of Raw Aluminum Source (%) |
|---|---|---|---|---|
| M1 | 9.5 | 0.28 | 19.81 | 97.0 |
| M2 | 8.1 | 0.29 | 24.60 | 96.5 |
| M3 | 7.2 | 0.30 | 32.84 | 97.2 |
| M4 | 8.0 | 0.25 | 20.11 | 95.9 |
| M5 | 7.9 | 0.27 | 19.85 | 96.7 |
| M6 | 9.5 | 0.28 | 19.81 | 97.0 |
| M7 | 6.5 | 0.23 | 20.15 | 92.8 |
| D1 | 4.4 | 0.20 | 20.58 | 90.3 |
| D2 | 4.3 | 0.21 | 20.44 | 91.1 |

**Table 2. Results of using the catalysts obtained in examples in the transalkylation of polycyclohexylbenzene and benzene**

| Catalyst No. | Conversion rate of dicyclohexylbenzene, wt% | Selectivity of cyclohexylbenzene, wt% |
|---|---|---|
| C1 | 70.2 | 99.5 |
| C2 | 68.5 | 99.0 |
| C3 | 67.3 | 99.1 |
| C4 | 67.2 | 99.0 |
| C5 | 68.6 | 99.2 |
| C6 | 67.7 | 99.4 |
| C7 | 65.4 | 98.5 |
| C8 | 62.1 | 93.4 |
| C9 | 63.5 | 92.8 |

The inventive examples are only detailed descriptions of the technical solutions of the present invention, but the present invention is not limited to the above examples, that is, the present invention can be implemented without relying on the steps described in the above examples. In summary, any modifications made by those skilled in the art to the present invention, including replacement of the raw materials and additives described in the present invention, selection of specific implementation manners, and the like, fall within the protection scope and disclosure scope of the present invention.

## Claims

1. An MWW-type molecular sieve, wherein a weight ratio of framework aluminum (Al_{[F]}) to extra-framework aluminum (Al_{[EF]}) ranges from 5.0:1 to 16.0:1, and preferably from 6.5:1 to 10.5:1.

2. The molecular sieve according to claim 1, wherein the molecular sieve has a weight ratio of aluminum in semi-supercages on its outer surface (Al₍₆₁₎) to the framework aluminum (Al_{[F]}) of from 0.18:1 to 0.60:1, and preferably from 0.22:1 to 0.50:1.

3. The molecular sieve according to claim 1 or 2, wherein the molecular sieve has a SiO₂/Al₂O₃ molar ratio of from 10 to 60, and preferably from 15 to 50.

4. The molecular sieve according to claim 1, wherein the molecular sieve has a pore volume of micropores of from 0.15 to 0.25 cm³/g, a pore volume of mesopores of from 0.35 to 0.55 cm³/g, and a specific surface area of from 400 to 550 m²/g

5. The molecular sieve according to claim 1, wherein the molecular sieve is an MWW-type silica-alumina molecular sieve having a 12-membered ring channel structure, and preferably an MCM-22 molecular sieve.

6. A method for preparing the molecular sieve according to any one of claims 1 to 5, comprising:
(a) contacting a silicon source, an aluminum source, an alkali source, water, a template agent, and a stabilizer to obtain a mixture A;
(b) crystallizing the mixture A to obtain the molecular sieve.

7. The method according to claim 6, wherein in step (a), the silicon source is one or more of tetraethoxysilane, alkaline silica sol, white carbon black, sodium silicate, and silicic acid; and/or
the aluminum source is one or more of sodium aluminate, pseudo-boehmite, aluminum isopropoxide, aluminum sulfate, and aluminum chloride; and/or
the alkali source is one or more of sodium hydroxide and potassium hydroxide; and/or
the template agent is one or more of hexamethyleneimine, piperidine, ethylenediamine, cyclohexylamine, and dimethylcyclohexylamine.

8. The method according to claim 6, wherein in step (a), the stabilizer is at least one amide compound of the following formula: 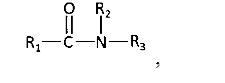 wherein R₁, R₂ and R₃ are each independently selected from the group consisting of hydrogen, C1-C15 alkyl, C5-C15 cycloalkyl, and C4-C15 nitrogen-containing organic groups, and preferably at least one of R₁, R₂ and R₃ is a C5-C10 cycloalkyl or a C4-C9 nitrogen-containing heterocyclic organic group, for example, R₁, R₂ and R₃ are each independently selected from the group consisting of hydrogen, ethyl, cyclohexyl, piperidinyl, pyridinyl, pyrimidinyl, methyl piperidinyl, methyl pyridinyl and methyl pyrimidinyl, and further preferably, the stabilizer is at least one selected from the group consisting of 4-piperidinecarboxamide, N-cyclohexylformamide, N-ethylformamide, 2-pyridinecarboxamide, and 6-methylnicotinamide.

9. The method according to any one of claims 6 to 8, wherein in step (a), a molar ratio of the silicon source in terms of SiO₂ to the aluminum source in terms of Al₂O₃ to the alkali source to the water to the template agent to the stabilizer is 1 : (0.01~0.10) : (0.02~0.50) : (8~25) : (0.05~0.5) : (0.01~0.30), and preferably 1 : (0.01~0.07) : (0.05~0.30) : (10~20) : (0.1~0.5) : (0.02~0.10).

10. The method according to claim 6, wherein conditions for the crystallizing in step (b) include: dynamically crystallizing under closed conditions, a crystallization temperature of 90 to 190 °C, and a crystallization time of 20 to 120 hours.

11. The method according to claim 6, wherein a utilization rate of the raw aluminum source is from 91.0 % to 99.0 %, and preferably from 93.0 % to 98.0 %.

12. A transalkylation catalyst, comprising the molecular sieve according to any one of claims 1 to 5 or the molecular sieve obtained by the method according to any one of claims 6 to 11, and an optional binder.

13. A method for preparing cyclohexylbenzene, comprising contacting a polycyclohexylbenzene and benzene in the presence of the catalyst of claim 12 to perform a transalkylation reaction to obtain a reaction effluent comprising cyclohexylbenzene, and separating cyclohexylbenzene from the reaction effluent.

14. A hydroalkylation catalyst, comprising the molecular sieve according to any one of claims 1 to 5 or the molecular sieve obtained by the method according to any one of claims 6 to 11, a hydrogenation metal and an optional binder.

15. A method for preparing cyclohexylbenzene via hydroalkylation of benzene, comprising contacting hydrogen and benzene with the hydroalkylation catalyst of claim 14 under hydroalkylation conditions to produce a reaction effluent comprising cyclohexylbenzene, and separating cyclohexylbenzene from the reaction effluent.
